Europäisches Patentamt

European Patent Office

Office européen des brevets

(11) Veröffentlichungsnummer: **0 295 435**
**A2**

## (12) EUROPÄISCHE PATENTANMELDUNG

(21) Anmeldenummer: 88107784.6

(22) Anmeldetag: 14.05.88

(51) Int. Cl.4: **C07C 31/20 , C07C 29/17**

(30) Priorität: 23.05.87 DE 3717405
16.10.87 DE 3735108

(43) Veröffentlichungstag der Anmeldung:
21.12.88 Patentblatt 88/51

(84) Benannte Vertragsstaaten:
BE CH DE FR GB IT LI NL

(71) Anmelder: **BASF Aktiengesellschaft**
**Carl-Bosch-Strasse 38**
**D-6700 Ludwigshafen(DE)**

(72) Erfinder: **Mueller, Herbert, Dr.**
**Carostrasse 53**
**D-6710 Frankenthal(DE)**
Erfinder: **Toussaint, Herbert, Dr.**
**Knietschstrasse 11**
**D-6710 Frankenthal(DE)**
Erfinder: **Schossig, Juergen, Dr.**
**Raiffeisenstrasse 16**
**D-6701 Fussgoenheim(DE)**

(54) **Verfahren zur Herstellung von Alkanolen aus Alkinolen.**

(57) Verfahren zur Herstellung von Alkanolen durch katalytische Hydrierung von Alkinolen mit 3 bis 8 C-Atomen, bei dem man eine Lösung des Alkinols in einem $C_1$-$C_8$-Alkanol mit einem Alkinolgehalt von über 5 Gew.%, einem Wassergehalt von höchstens 10 Gew.% und einem Gehalt an dem Alkanol von 20 bis 85 Gew.%, bei Temperaturen von 100 bis 240 °C und Drücken von über 10 bar und einem pH-Wert von größer als 6 hydriert.

EP 0 295 435 A2

## Verfahren zur Herstellung von Alkanolen aus Alkinolen

Diese Erfindung betrifft ein Verfahren zur Herstellung von Alkanolen durch katalytische Hydrierung von Alkinolen.

Bekanntlich lassen sich Alkanole, wie Propanol oder Butandiol-1,4, durch katalytische Hydrierung von Alkinolen, wie Propin-2-ol-1 oder Butin-2-diol-1,4, herstellen. Nach den in der DE-OS 25 36 273 oder der US-PS 4 153 578 beschriebenen Verfahren verwendet man z.B. Nickel- oder Kobaltkatalysatoren, die entweder als Raney-Metalle oder als Mischkatalysatoren eingesetzt werden. Man hat für die Hydrierung von Acetylenalkoholen auch Trägerkatalysatoren, die neben Nickel noch Kupfer, Aluminium und Mangan enthalten, vorgeschlagen (s. DE-OS 21 45 297, US-PS 3 449 445 und DE-OS 20 04 611).

Mit dem in der DE-OS 25 36 273 beschriebenen Katalysator, der neben Nickel noch Kupfer, Mangan und Molybdän enthält, werden zwar befriedigende Standzeiten bei der Hydrierung von Butindiol-1,4 zu Butandiol-1,4 erreicht. Um ein Butandiol zu erhalten, das hohen Reinheitsanforderungen entspricht, müssen jedoch verhältnismäßig hohe Wasserstoffdrücke und Temperaturen gewählt werden, da es nur so gelingt, die sich bei vielen Anwendungen nachteilig auswirkenden Carbonylverbindungen und deren Derivate soweit reduzierend zu entfernen, daß ihr schädlicher Einfluß toleriert werden kann. Eine derartige Verschärfung der Reaktionsbedingungen fördert jedoch die unerwünschte Bildung von Butanol und vermindert die Ausbeute; gleichzeitig erhöht sich der Anfall des unerwünschten Nebenproduktes 2-Methylbutandiol (s. US-PS 4 153 578). Schließlich macht es auch Schwierigkeiten, den die aktiven Metalle enthaltenden Katalysator reproduzierbar mit gleicher Aktivität und Lebensdauer herzustellen.

Bei den technisch ausgeübten Alkinol-Hydrierungen werden bevorzugt Nickel und Kobalt enthaltende Katalysatoren verwendet. Da sowohl nickel- als auch kobalthaltige Stäube als Krebs erzeugende Arbeitsstoffe erkannt wurden, gestaltet sich der Umgang mit nickel- bzw. kobalthaltigen Festbettkatalysatoren sehr schwierig und kostspielig. Der Einsatz solcher Katalysatoren ist heute nur noch dann vertretbar, wenn sie über sehr lange Zeiträume die ursprüngliche Gebrauchsfähigkeit, z.B. in Bezug auf Aktivität, Selektivität und Festigkeit beibehalten, und ein häufiger Katalysatorwechsel entfällt. Man hat deshalb auch schon den Gebrauch suspendierter Nickelkatalysatoren vorgeschlagen (US-PS 4 599 466), bei deren Handhabung diese Schwierigkeiten weitgehend vermieden werden.

Von großer technischer Bedeutung ist die Hydrierung von Butin-2-diol-1,4 zu Butandiol-1,4. Dabei werden die rohen wäßrigen Butindiol-Lösungen eingesetzt, wie sie bei der Synthese des Butandiols nach Reppe aus Acetylen und Formaldehyd erhalten werden (US 4 371 723).

Als eine besonders schwierige Aufgabe bei der Hydrierung dieser rohen, wäßrigen Butindiol-Lösungen hat sich die Entfernung von Carbonyl-Verbindungen bzw. deren Acetale erwiesen. So enthalten diese Butindiol-Lösungen herstellungsbedingt nicht zu vernachlässigende Mengen an Formaldehyd, durch die die Hydrierung des Butindiols gestört und unerwünschte Nebenprodukte gebildet werden. Es wurde deshalb schon vorgeschlagen, den Formaldehyd vor der Hydrierung durch Destillation aus den Roh-Butindiol-Lösungen zu entfernen (vgl. US-PS 3 449 445, Spalte 2, Zeilen 57-60) oder durch Alkalizugabe zu unschädlichem Polymerisat umzusetzen (US-PS 4 180 687). Formaldehyd hat nicht nur nachteilige Wirkungen auf den Hydrierkatalysator, er bewirkt auch, daß aus Butindiol bei der Hydrierung 2-Methylbutandiol-1,4 gebildet wird. Dieses Produkt ist im Rein-Butandiol unerwünscht, weil es auch in den Butandiol-Folgeprodukten (wie im N-Methylpyrrolidon) als Quelle für kaum abtrennbare Verunreinigungen auftritt. Es läßt sich zudem destillativ kaum vom gewünschten reinen Butandiol-1,4 abtrennen. Deshalb wird in der US-PS 4 153 578 vorgeschlagen, die Butindiol-Lösung zunächst bei niederen Drücken und niederen Temperaturen weitgehend zu hydrieren und erst in einer zweiten Stufe an einem speziellen molybdänhaltigen Raney-Nickel zu Ende zu hydrieren. Dadurch gelingt es, den Methylbutandiol-Gehalt im Butandiol von normalerweise 1,5 bis 2 % auf bestenfalls 0,3 bis 0,6 % zu reduzieren. Ein solches Verfahren ist aber aufwendig und kostspielig.

Die genannten Butindiol-Lösungen enthalten nicht nur freien Formaldehyd, sondern auch mehr als 0,2 Gew.% Polyoximethylene. Diese können die aktiven Katalysatoroberflächen belegen und damit die Hydrierung beeinträchtigen. Sie erscheinen außerdem bei der Reindestillation des Butandiols als wertloser Rückstand, dessen Beseitigung nach der destillativen Aufarbeitung der Butandiol-Roh-Lösung Kosten verursacht. Es hat sich auch gezeigt, daß niedere Polyoximethylene im Rein-Butandiol als Verunreinigungen enthalten sein können, wenn die destillative Aufarbeitung nicht mit einem sehr hohen technischen Aufwand durchgeführt wird. Solches Butandiol ist z.B. ungeeignet, um für die Herstellung von Tetrahydrofuran (THF) eingesetzt zu werden. Die Polyoximethylene reduzieren nämlich die Aktivität der Dehydratisierungskatalysatoren. THF ist ein sehr wichtiges Folgeprodukt von Butandiol-1,4. Ein bedeutender Anteil THF wird für die Herstellung von Polytetrahydrofuran herangezogen. Da die Reinheit des THF nicht nur die erzielbaren Molekulargewichte bei der Polymerisation, sondern auch die Polymerisationsgeschwindigkeit, die Aktivität und Lebensdauer der Polymerisationskatalysatoren und insbesondere die Farbe des Polymeren beeinflußt, werden an die Reinheit des THF hohe Anforderungen gestellt. So ist z.B. vorgeschlagen worden, THF vor der Polymerisation einer Behandlung mit Laugen zu unterwerfen, durch die die Farbbildung im Polymeren unterdrückt wird (US-PS 4 544 774).

Es bestand deshalb ein Bedarf, ein Verfahren für die Hydrierung von Alkalinolen, wie Butindiol-1,4, zu finden, das es ermöglicht, ein besonders gut geeignetes Ausgangsprodukt für die Herstellung von THF zu gewinnen. Dabei sollte die Hydrierung des Butindiols auf besonders wirtschaftliche Weise bei hohem Durchsatz und niedrigen Katalysatorkosten erfolgen. Neben der geforderten Reinheit des Butandiols war auch eine hohe Ausbeute zu erreichen. Wünschenswert war es auch, daß bei dem Hydrierverfahren im rohen Butin-2-diol-1,4 vorhandene Nebenprodukte, wie Hydroxibutyraldehyd oder davon abgeleitete Acetale und Acetale des Butindiols ebenfalls in Butandiol-1,4 übergeführt werden. Desweiteren war anzustreben, daß die in den rohen Alkindiol-Lösungen vorhandenen Formaldehydpolymere (Polyoximethylene) durch Hydrierung gespalten und in Methanol übergeführt werden. Hierdurch würde erreicht, daß man aus den sonst unbrauchbaren Polymeren das Wertprodukt Methanol gewinnt, wodurch gleichzeitig die Reindestillation des Butandiols entlastet wird.

Ein Teil dieser Aufgaben wurde bereits durch das in der US-Patentschrift 4 599 466 beschriebene Verfahren gelöst, und zwar dadurch, daß man die rohen wäßrigen Butindiol-Lösungen nach Zugabe von Nickelsalzen organischer Säuren bei Temperaturen über 190°C hydriert. Die dabei entstehenden Katalysatoren sind in der Anfangsphase außerordentlich selektiv, bilden nur wenig Nebenprodukte und liefern eine hohe Ausbeute. Diese guten Eigenschaften lassen aber nach längeren Betriebszeiten nach. Während die Beeinträchtigung der Selektivität anfangs nur gering ist, verstärkt sie sich beim weiteren Betrieb.

Nach dem Verfahren der Erfindung, das die gestellten Aufgaben in hohem Maße erfüllt und die genannten Nachteile vermeidet, werden Alkinole mit 3 bis 8 C-Atomen bei höheren Temperaturen und Drücken dadurch katalytisch hydriert, daß man eine Lösung des Alkinols in einem Alkanol, das 1 bis 8 C-Atome aufweist, mit einem Alkinolgehalt von über 5 Gew.%, einem Wassergehalt von höchstens 10 Gew.% und einem Gehalt an dem Alkanol von 20 bis 85 Gew.%, bei Temperaturen von 100 bis 240°C und Drücken von über 10 bar und einem pH-Wert von größer als 6 hydriert.

Als Alkinole sind für die erfindungsgemäße Hydrierung solche mit 3 bis 8 C-Atomen geeignet, die z.B. 1 bis 2 Hydroxylgruppen und 1 bis 2 Dreifachbindungen enthalten. Beispielsweise seien Propin-2-ol-1, Butin-2-diol-1,4, Hexin-3-diol-2,5 oder 2,5-Dimethylhexin-3-diol-2,5 genannt. Die Hydrierung von Butin-2-diol-1,4 zum Butandiol-1,4 ist von besonderem technischen Interesse.

Die genannten Alkinole werden als Lösungen in einem Alkanol, das 1 bis 8 C-Atome aufweist, hydriert. Die Ausgangslösungen haben einen Alkinolgehalt von über 5 Gew.%. Beispielsweise liegt die Konzentration des Alkinols in der Lösung bei 10 bis 80, vorzugsweise bei 30 bis 70 Gew.%. Die Ausgangslösungen haben einen Wassergehalt von höchstens 10 Gew.%. Vorzugsweise liegt der Wassergehalt der Lösungen unter 5 Gew.%. Der Alkanolgehalt der Ausgangslösungen beträgt 20 bis 85, vorzugsweise 30 bis 70 Gew.%. Als Alkanole der genannten Art kommen Alkanole mit 1 bis 2 Hydroxylgruppen, vorzugsweise einwertige geradkettige oder verzweigte Alkanole mit 1 bis 5 C-Atomen, wie Methanol, Ethanol, Propanol, Butanol, tert.-Butanol in Betracht, von denen Methanol besonders bevorzugt ist. Glykole, wie Ethylenglykol oder Butandiol-1,4 eignen sich zwar als Lösungsmittel für das erfindungsgemäße Verfahren ebenfalls, verdienen aber wegen der hohen Siedepunkte keine Bevorzugung.

Die alkoholischen Ausgangsgemische können außerdem durch die Synthese des Alkinols bedingte Verunreinigungen enthalten. Solche Verunreinigungen, deren Konzentration in den Ausgangsgemischen z.B. bis zu 4 Gew.% beträgt, sind z.B. Aldehyde, wie Formaldehyd und dessen Polymere, Acetale oder auch nicht oder schwer flüchtige Alkin-Funktionen enthaltende Verbindungen sowie Alkalisalze von niedermolekularen organischen Carbonsäuren, wie Ameisen- oder Essigsäure. Es können auch vom verwendeten Ethinylierungskatalysator stammende Kieselsäure und Alkali- bzw. Erdalkalisilikate enthalten sein oder Formiate, die aus Formaldehyd durch eine Canizzaro-Reaktion entstanden sind. Natriumacetat ist z.B. vorhanden, wenn man bei der Ethinylierung zur pH-Kontrolle Puffersubstanzen eingesetzt hat.

Die für das erfindungsgemäße Verfahren benötigten Gemische, die die alkoholischen Lösungen der Alkinole enthalten, werden z.B. dadurch zur Verfügung gestellt, daß man die bei der bekannten (s. Ullmanns Enzyklopädie der Technischen Chemie (1953), Bd. III, Seiten 109-119, Bd. IV, Seiten 754-757, DE-AS 24 21 407 und DE-OS 25 36 273) und großtechnisch ausgeübten Synthese von Butin-2-diol-1,4 aus Acetylen und wäßrigem Formaldehyd erhältlichen rohen wäßrigen Butindiol-Lösungen bis auf einen Restgehalt an Wasser von höchstens 10, vorzugsweise höchstens 5 Gew.% eindampft. Man destilliert das Wasser unter Normaldruck oder im Vakuum ab. Die so erhältlichen Konzentrate, die noch die höher als Wasser siedenden Verunreinigungen, wie Carbonyl- oder Acetal-Verbindungen, höhermolekulare Alkinole, polymeren Formaldehyd, und die oben erwähnten nicht flüchtigen Rückstandssubstanzen enthalten können, werden dann in dem Alkanol aufgenommen, wobei man soviel Alkanol zugibt, daß die oben beschriebenen Ausgangslösungen erhalten werden.

Eine für die Herstellung von Butandiol-1,4 besonders gut geeignete Ausgangslösung enthält z.B. die folgenden Bestandteile: 30 bis 70 Gew.% Butindiol-1,4, 30 bis 70 Gew.% Methanol, 0,2 bis 5 Gew.% Wasser und 0,2 bis 5 Gew.% synthesebedingte Verunreinigungen, wie Formaldehyd, Polyoximethylen, Acetale, nicht flüchtige organische Substanzen, Natriumformiat und Propin-2-ol-1. Normalerweise besitzt eine solche Ausgangslösung einen so hohen Alkaligehalt, daß sie einen pH-Wert von größer als 6 aufweist.

Bevorzugt sind pH-Werte von 7 bis 9. Wird der pH-Wert von 10 überschritten, so empfiehlt es sich, den pH-Wert durch Zugaben von Puffersubstanzen zu erniedrigen. Den pH-Wert mißt man z.B. mit einer Glaselektrode unter Zusatz von 50 Gew.% Wasser.

Als Katalysatoren sind alle für katalytische Hydrierungen gebräuchliche Katalysatoren, wie Platin, Palladium, Ruthenium, Nickel, Kobalt oder Kupfer geeignet. Die Hydrierkatalysatoren können auch mehrere dieser katalytisch wirkenden Metalle enthalten. Bevorzugt sind Katalysatoren, deren katalytisch wirkende Komponente zu über 50 Gew.%, vorzugsweise über 99 Gew.% aus Nickel besteht, wie Raney-Nickel oder Nickel, das durch den Zerfall von Nickel-O-Komplexen oder Nickel-Formiat gebildet wird. Die Nickel-Katalysatoren können auch Zusätze von anderen hydrierend wirkenden Metallen, wie Palladium, Rhodium, Ruthenium, Kobalt und Kupfer enthalten. Reine Nickelkatalysatoren sind aber bevorzugt. Nach einer besonders bevorzugten Ausführungsform des Verfahrens wird der Katalysator als Nickelsalz einer organischen Carbonsäure mit mehr als einem Kohlenstofatom in die alkoholische Lösung des zu hydrierenden Alkinols eingebracht. Im einfachsten Falle setzt man Nickel-Acetat in einer Konzentration von z.B. 0,05 bis 0,005 Gew.%, insbesondere von 0,03 bis 0,01 Gew.-% Nickel ein. Das katalytisch wirksame Nickel bildet sich dann im Reaktionsmedium unter den Reaktionsbedingungen von selbst. Höhere Konzentrationen an Nickel sind für die Umsetzung unschädlich, verringern aber die Wirtschaftlichkeit des Verfahrens. Geeignete Nickelsalze, die sich in den alkoholischen Lösungen der Alkinole in ausreichender Konzentration lösen, sind z.B. Nickel-Acetat, Nickel-Formiat, Nickel-Butyrat und z.B. Nickel-2-ethylhexanoat.

Man kann auch Trägerkatalysatoren verwenden, in denen das aktive Hydriermetall auf einem an sich üblichen Trägermaterial aufgebracht ist. Häufig, weil für die Temperaturführung günstig, werden die Katalysatoren in suspendierter Form, z.B. in gerührten Reaktoren oder in Blasensäulen eingesetzt. Es ist aber auch möglich, die Katalysatoren in einem Festbett anzuordnen und das zu hydrierende Substrat nach der Sumpf- oder Rieselfahrweise zu hydrieren. Die Sumpffahrweise ist bevorzugt. Beim erfindungsgemäßen Einsatz von im Festbett festangeordneten Katalysatoren wurde festgestellt, daß z.B. Trägerkatalysatoren auf Aluminiumoxid- oder Kieselsäurebasis eine Lebensdauer von einem Jahr und länger aufweisen, ehe sie an Aktivität oder Festigkeit verlieren. Dies ist deshalb so überraschend, weil solche Trägerkatalysatoren durch Alkinol-Lösungen, wie man sie nach technisch geläufigen Verfahren für die Hydrierung verwendet, schnell zerstört werden und nur eine Lebensdauer von 1 bis 3 Monaten erreichen.

Der Einsatz von Festbettkatalysatoren bietet den Vorteil, daß die Katalysatoren stationär sind und aus dem erhaltenen Reaktionsprodukt nicht abgetrennt werden müssen. Dennoch wird man in vielen Fällen suspendierte Katalysatoren bevorzugen, da sich diese aus dem Reaktionsaustrag, vor allem wenn es sich um Nickel-Katalysatoren handelt, sehr leicht z.B. durch Filtration über porösen Sinterwerkstoff oder Magnetfilter quantitativ abtrennen lassen. Die Arbeitsweise, bei der organische Nickel-Salze als Vorläufer für die Katalysatoren eingesetzt werden, hat den Vorteil, daß die anzuwendenden Konzentrationen an aktivem Metall sehr niedrig sind, so daß man sie von den Filtern in einfacher Weise in gelöster Form als Salze entfernen und erneut für die Hydrierung in den Kreislauf zurückführen kann. Da diese Operationen im geschlossenen System mit feuchtem Metall ausgeführt werden, wird eine Gefährdung des Bedienungspersonals durch die Metalle ausgeschlossen.

Man hydriert die Ausgangsgemische in Gegenwart der Hydrierungskatalysatoren bei Temperaturen von 110 bis 240°C, vorzugsweise bei 110 bis 210°C. Wird als Katalysator das Nickelsalz einer organischen Säure zu der Alkinollösung gegeben, so hydriert man bei Temperaturen über 150°C, vorteilhaft bei 170 bis 240°C. Der Wasserstoffpartialdruck sollte bei der Hydrierung zwischen 10 bis 350 bar, vorzugsweise zwischen 50 und 250 bar, insbsondere zwischen 150 und 250 bar, liegen. Drücke, die über den bevorzugten Bereich hinausgehen, bringen gemessen am Aufwand keinen merklichen Vorteil. So läßt sich z.B. aus einer methanolischen Ausgangslösung mit einem Butindiol-Gehalt von 50 Gew.% an 0,04 Gew.-% Nickel, das als Nickelacetat in die Reaktion eingebracht wurde, in einer Stufe bei Temperaturen um 200°C und einem Gesamtdruck von 250 bar ein Butandiol höchster Reinheit mit quantitativer Selektivität herstellen. Die Konzentration des Hydrierkatalysators richtet sich nach der gewünschten Reaktionsgeschwindigkeit bei der gewählten Reaktionstemperatur. Sie liegt im allgemeinen mit z.B. 0,01 bis 0,1 Gew.% (vergl. mit den obengenannten anderen Werten) wesentlich niedriger als bei der sonst geübten Hydrierarbeitsweise.

Man führt das neue Verfahren chargenweise oder kontinuierlich durch. Bei der besonders vorteilhaften kontinuierlichen Verfahrensführung, die man z.B. in einem Rührreaktor oder einem Umlaufreaktor durchführt, wird die Hydrierwärme zweckmäßigerweise dazu verwendet, den Frischzulauf z.B. auf eine Eingangstemperatur von 90 bis 180°C aufzuheizen, ehe er in den Reaktor eintritt. Das Aufheizen erfolgt z.B. über einen außen liegenden Wärmetauscher, der vom rückgeführten Reaktionsgemisch durchströmt wird. In dem Maße, in dem die Hydrierung längs der Reaktionszone fortschreitet, erhitzt sich das Reaktionsgemisch auf z.B. die Reaktionstemperatur von 120°C und höher. Die Endtemperatur wird in diesem Falle überwiegend von der Menge des Frischzulaufes und der z.B. für die Dampfgewinnung verwendeten Menge der Hydrierwärme bestimmt.

Wird nicht kontinuierlich hydriert, so empfiehlt sich die sogenannte "halb-kontinuierlich" durchgeführte Hydrierung. Dabei wird der Katalysator in einem kleinen Teil des hydrierten Reaktionsgemisches suspendiert oder gelöst vorgelegt. Dann wird in das Reaktionsgefäß bei Reaktionstemperatur und Reaktionsdruck Butindiol-Lösung kontinuierlich in dem Maße, wie die Hydrierung stattfindet, eingeleitet. Auf diese Weise wird vermieden, daß das Butindiol im zeitlichen Verlauf der Hydrierung in überhöhter Konzentration im Reaktionsvolumen auftritt.

Das im Reaktionsgemisch suspendierte Hydriermetall trennt man nach der Hydrierung, z.B. nach an sich üblichen physikalischen Trennmethoden, wie Zentrifugieren, Sedimentieren oder Filtrieren ab. Die Katalysatoren können erneut in den Reaktionsraum zurückgeführt werden. Sie erhalten ihre ursprüngliche Aktivität über sehr lange Reaktionszeiten bei. Verwendet man als Katalysator ein organisches Nickel-Salz, das sich unter den Reaktionsbedingungen von selbst aktiviert, so ist die notwendige Nickelkonzentration so gering, daß sich die Abtrennung des Katalysators zum Zwecke der Rückführung nicht lohnt. Vielmehr wird man in diesem Falle das durch die Abtrennmethode gewonnene Metall zweckmäßig in einer organischen Säure lösen und erneut für die Hydrierung dem Frischzulauf zusetzen.

Nach dem neuen Verfahren erhält man die Alkanole aus den Alkinolen in außerordentlich hoher Reinheit. Die Selektivität beträgt praktisch 100 %. So ist z.B. das erfindungsgemäß hergestellte Butandiol frei von Carbonyl-Funktionen oder Spuren von olefinisch ungesättigten Verbindungen. Es hat eine Farbzahl, die deutlich unter 50 APHA liegt. Der Gehalt an Methylbutandiol liegt bereits im Roh-Produkt bei unter 0,1 Gew.%, vorzugsweise bei unter 0,05 Gew.%. Der Rückstandswert der rohen Butandiol-Lösung beträgt höchstens 40 % des Rückstandswertes der zur Hydrierung verwendeten rohen Butindiol-Lösung. Wegen seiner hohen Reinheit ist das erhaltene Butandiol besonders für die Herstellung von monomerem THF geeignet, das z.B. zur Herstellung von Polytetrahydrofuran mit niedriger Farbzahl verwendet werden soll. Wegen der hohen Lebensdauer der Katalysatoren kann man das Verfahren auch mit sehr guten Ergebnissen mit einem stationären Festbettkatalysator entweder in Sumpf- oder Rieselfahrweise anwenden, ohne daß man Gefahr läuft, wegen nachlassender Aktivität häufigen Katalysatorwechsel durchführen zu müssen. Das Verfahren zeichnet sich durch hohe Wirtschaftlichkeit aus, weil die Reaktoren praktisch unbegrenzte Laufzeit besitzen und keiner Reinigungsoperation unterworfen werden müssen. Da die Hydrierung bei Temperaturen von über 180°C durchgeführt werden kann, kann man die bei der Reaktion entstehende Hydrierenthalpie bei hohem Temperaturniveau gewinnen. Die Wirtschaftlichkeit des Verfahrens wird dadurch weiter erhöht. Der Durchsatz, der, gemessen als Kilogramm-Wertprodukt pro Liter Reaktionsraum mal Stunde, erzielt wird, ist bei gleichem Druck und gleicher Temperatur sehr viel höher als bei den bekannten Verfahren.

Daß die hohen Ausbeuten ohne vorherige Abtrennung von Verunreinigungen aus den rohen Lösungen erzielt werden, ist überraschend, da Lösungsmittel, die mit den erfindungsgemäß verwendeten Alkoholen vergleichbare Eigenschaften besitzen, wie Tetrahydrofuran oder Diethylether, die hier beobachtete ausbeutesteigernde Wirkung bei der Hydrierung nicht zeigen. Da durch die Verwendung der Alkohole als Lösungsmittel die Konzentration an zu Carbonylfunktionen dehydrierbaren Hydroxylfunktionen im Reaktionsgemisch sehr hoch ist, waren vielmehr unerwünschte Nebenreaktionen nach vorangegangener Dehydrierung, wie Carbonylkondensation oder Acetalbildung zu erwarten. Es war auch nicht vorhersehbar, daß die in den Ausgangslösungen enthaltenen bei der Hydrierung als nachteilig erkannten polymeren Alkinole, deren genaue Struktur nicht bekannt ist, bei der erfindungsgemä-

Ben Hydrierung in die gesuchten Alkanole umgewandelt werden, so daß eine Endausbeute, bezogen auf die Ausgangsmenge an Alkinol, von über 100 Mol% erzielt werden kann.

Beispiel 1

Für die kontinuierliche Hydrierung von Butindiol-1,4 wurde von einer rohen wäßrigen Butindiol-1,4-Lösung ausgegangen, die durch Umsetzung von Acetylen mit wäßrigem Formaldehyd erhalten wurde. Sie bestand aus 1 bis 2 Gew.% Methanol, 1 bis 2 Gew.% Propinol, 0,9 Gew.% Formaldehyd, 0,6 Gew.% Formaldehyd-Polymeren, 39 Gew.% Butindiol-1,4, 55 bis 60 Gew.% Wasser sowie aus 2 bis 4 Gew.% nicht identifizierter Acetale und nicht flüchtigen Anteilen. Diese Lösung wurde an einem Rotationsfilmverdampfer bei einer Verdampfertemperatur von 140 bis 150°C und Normaldruck von der Hauptmenge des Wassers durch Destillation befreit. Der heiße Sumpf des Filmverdampfers wurde danach durch Entspannungsverdampfung bei 20 bis 30 mbar bis zu einem Wassergehalt von 0,3 bis 0,8 Gew.% getrocknet. Das im Butindiol enthaltene Methanol, Formaldehyd und Propinol wurden dabei weitgehend mit dem Wasser abdestilliert.

Der Destillationssumpf wurde in der gleichen Gewichtsmenge Methanol gelöst. Die so erhaltene methanolische Lösung hatte folgende Zusammensetung: 47 Gew.% Butindiol-1,4, 0,3 Gew.% Wasser, 50 Gew.% Methanol, 0,15 Gew.% Formaldehyd und 2,5 Gew.% an sonstigen Nebenproduken oder Verunreinigungen. Die methanolische Butindiol-Lösung wurde mit dem Katalysator Nickel-Acetat versetzt. Die Menge des Nickel-Acetats in der methanolischen Lösung wurde so gewählt, daß die Konzentration an reinem Nickel 0,05 Gew.% betrug. Durch Zusatz von 3 ml 30 %iger Natrium-Methylat-Lösung in Methanol pro Kilogramm der nickel-acetat-haltigen methanolischen Butindiol-Lösung wurde der für das Verfahren günstige pH-Bereich von 8 bis 9 eingestellt. Für die Bestimmung des pH-Wertes verwendete man eine Glaselektrode, die in eine Lösung aus gleichen Teilen Wasser und der methanolischen nickel-acetat-haltigen Butindiol-Lösung eingetaucht wurde.

Die Hydrierung wurde in einem senkrecht stehenden Reaktionsrohr mit 1 000 Volumenteilen Reaktionsinhalt durchgeführt. Das Verhältnis des Durchmessers zur Länge des Reaktionsrohres betrug 1 : 40. Das Reaktionsrohr enthielt keine Einbauten. Es wurde von unten nach oben mit Zulauf beschickt, der am oberen Ende in einen Hochdruckabscheider abfloß. Die Wasserstoffzufuhr erfolgte ebenfalls am unteren Ende des Reaktionsrohres. Der Wasserstoffdruck auf dem Hochdruckabscheider und dem Reaktionsrohr betrug 250 bar. Aus der Gasphase des Hochdruckabscheiders wurden stündlich 100 bis 1 000 Normalvolumenteile Wasserstoff als Abgas entnommen. Der Zulauf wurde auf 180 bis 190°C vorgewärmt und in das Reaktionsrohr kontinuierlich eingepumpt. Die freiwerdende Hydrierwärme wurde mittels einer innen liegenden Rohrschlange nach außen über einen Wärmetauscher abgeführt (die Wärme kann mit gleich gutem Erfolg auch durch direkte Kühlung mittels eines im Kreis geführten Teilstromes des Reaktionsproduktes in einem außen liegenden Kühler abgeführt werden). Im Reaktionsrohr wurde eine mittlere Temperatur von 185 bis 195°C eingehalten. Dem Reaktionsrohr wurde die zu hydrierende Lösung mit einer Zulaufgeschwindigkeit von 500 Gew.-Teilen pro Stunde zugeführt. Man erhielt stündlich 500 Gew.-Teile eines Reaktionsaustrages, für dessen Zusammensetzung, wasser- und methanolfrei gerechnet, die folgenden Bestandteile ermittelt wurden:

n-Butanol 0,4 Gew.%
Hydroxibutyraldehyd 0,005 Gew.%
Butandiol-1,4 99 Gew.%
2-Methylbutandiol-1,4 < 0,1 Gew.%
Acetale < 0,01 Gew.%
Summe Unbekannter 0,1 Gew.%
nichtflüchtige Anteile 0.4 Gew.%

Nach 90 Tagen Reaktionsdauer konnte die Frischzufuhr an Nickelacetat auf ca. 30 % der ursprünglichen Konzentration gesenkt werden, ohne daß die Hydrierung beeinträchtigt wurde. Das nach einigen Tagen Reaktionszeit im Austrag erscheinende metallische Nickel wurde mittels einer Filterkerze aus Sintermetall (Werkstoff: 14404, Filterfeinheit 65 Mikron), die zwischen Reaktionsrohr und Hochdruckabscheider eingebaut war, abfiltriert. Nach weieren 20 Tagen wurde der Reaktionsaustrag auf eine parallel geschaltete Metallfilterkerze umgeleitet und gelangte von dort in den Hochdruckabscheider. Nach Entfernen der ersten Metallfilterkerze wurde das darin befindliche Nickel-Metall in Nickelacetat zurückverwandelt, das in dieser Form erneut für die Hydrierung des Butindiols eingesetzt werden konnte. Abgesehen von mechanischen Verlusten wurde kein Katalysatorverbrauch festgestellt. Für die quantitative Abtrennung des metallischen Nickels aus dem Reaktionsaustrag kann auch eine Magnetabscheidung herangezogen werden.

Zur Abführung der Reaktionswärme durch direkte Kühlung wurde die 25-fache Menge des Reaktoreintrags dem Reaktorende entnommen, auf eine Temperatur von 160 bis 170°C abgekühlt und dem Reaktionsrohr erneut von unten zugeführt. Das Verhältnis des Kreislaufgutes zur Frischbeschickung betrug im Durchschnitt stets 25 : 1.

Beispiel 2

Aus einem Katalysator mit der Korngröße 2,5 bis 3,5 mm, der aus einer Nickel-Aluminium-Legierung bestand (42 Gew.% Ni und 58 Gew.% Al) wurde nach den Angaben der DE-OS 20 04 611 durch Behandlung mit einer 0,5 gew.%igen wäßrigen Natronlauge ca. 25 Gew.% des Aluminiums entfernt.

In der in Beispiel 1 beschriebenen Versuchsanordnung wurde die in Beispiel 1 eingesetzte methanolische Butindiol-Lösung hydriert. Dabei wurde der Zusatz des Nickelacetat-Katalysators unterlassen. Stattdessen wurde der Hydrierreaktor mit etwa 1 600 Gew.-Teilen des oben beschriebenen Nickel-Aluminium-Katalysators gefüllt. Durch Zugabe von 3 ml Natriummethylat-Lösung (30 gew.%ig in Methanol) pro kg Zulauf wurde der pH-Wert auf 8,5 gebracht. Der Hydrierreaktor wurde von unten nach oben mit 200 Gew.-Teilen der methanolischen Roh-Butindiol-Lösung beschickt. Durch Kreislaufführung eines Teiles des Reaktionsgutes im Verhältnis Kreislaufgut zu Frischzulauf wie 25 : 1 wurde die Temperatur im Hydrierreaktor zwischen 205 und 215°C gehalten. Der Wasserstoffdruck auf dem Hochdruckabscheider betrug 230 bar. Aus der Gasphase des Hochdruckabscheiders wurden stündlich 50000 Normalvolumenteile Wasserstoff als Abgas entnommen. Stündlich erhielt man 200 Teile Reaktionsaustrag, der sich, methanol- und wasserfrei gerechnet, aus folgenden Bestandteilen zusammensetzte:

n-Butanol          0,7 Gew.%
Hydroxibutyraldehyd          < 0,001 Gew.%
Butandiol-1,4          >98 Gew.%
2-Methylbutandiol-1,4          0,3 Gew.%
Acetale          0,3 Gew.%
nichtflüchtige Anteile          0,4 Gew.%

Nach 100 Tagen Reaktionsdauer wurde keine Veränderung des beschriebenen Hydrierergebnisses festgestellt.

Beispiel 3

Es wurde wie im Beispiel 1 beschrieben verfahren, wobei dem Reaktionsrohr stündlich 300 Teile einer 60 gew.%igen methanolischen Lösung von Hexin-3-diol-2,5, die soviel Nickelacetat in gelöster Form enthielt, daß der Nickelgehalt in der Lösung 0,05 Gew.% betrug, zugeführt wurde. Der gaschromatographisch untersuchte Reaktionsaustrag bestand (Wasser nicht berücksichtigt) zu etwa 99,5 Gew.% aus Hexandiol-2,5 und zu etwa 0,2 Gew.% aus Hexanol-2. Der Gehalt an nichtflüchtigen Bestandteilen im Rohprodukt lag bei unter 0,3 Gew.%.

Beispiel 4

Für die Hydrierung wurde die in Beispiel 1 beschriebene methanolische Lösung mit der Zusammensetzung: 47 Gew.% Butindiol-1,4, 0,3 Gew.% Wasser, 50 Gew.% Methanol, 0,15 Gew.% Formaldehyd und 2,5 Gew.% sonstige Nebenprodukte oder Verunreinigungen herangezogen. Die Hydrierung wurde in dem in Beispiel 1 beschriebenen Reaktionsrohr durchgeführt. Das Reaktionsrohr, das außer Thermoelementen zur Temperaturkontrolle bei der Hydrierung keine Einbauten enthielt, wurde mit 900 Volumenteilen eines Katalysators beschickt, der die folgenden als Oxide berechneten Bestandteile aufwies:

Nickeloxid:          21,5 %
Kupferoxid:          7,3 %
Mangan-II, III-oxid:          2 %
Phosphorpentoxid          1 %
Natriumoxid:          0,35 %
der Rest zu 100 bestand aus Kieselsäure.

Vor der Butindiol-Hydrierung wurde der Katalysator im Verlaufe von 48 Stunden bei Atmosphärendruck durch Überleiten von Wasserstoff bei steigenden Temperaturen von 180 bis 325°C aktiviert.

Für die Hydrierung wurde nun die methanolische Butindiollösung als Zulauf von unten nach oben durch das Reaktorrohr geleitet. Am oberen Ende des Reaktorrohres floß das Reaktionsgemisch frei ab, so daß die Hydrierung nach der sogenannten Sumpffahrweise ohne wesentlichen freien Gasraum durchgeführt wurde. Die Wasserstoffzufuhr erfolgte ebenfalls am unteren Ende des Reaktionsrohres. Der Wasserstoffdruck auf dem Hochdruckabscheider und dem Reaktionsrohr betrug 280 bar. Aus der Gasphase des Hochdruckabscheiders wurden stündlich 100 bis 1000 Normal-Volumenteile Wasserstoff als Abgas entnommen. Der Zulauf wurde auf 50°C vorgewärmt und in das Reaktionsrohr kontinuierlich eingepumpt. Die frei werdende Hydrierwärme wurde durch Außenkühlung und durch 5000 Volumen-Teile pro Stunde eines im Kreis geführten Teilstromes des Reaktionsproduktes, der in einem außen liegenden Kühler gekühlt wurde, abgeführt. Im Reaktionsrohr wurde eine mittlere Temperatur von 120 bis 130°C eingehalten. Das Reaktionsgemisch hatte einen pH-Wert von 7,8. Dem mit Katalysator gefüllten Reaktionsrohr wurde die zuhydrierende Lösung mit einer Zulaufgeschwindigkeit von 300 Gewichtsteilen pro Stunde zugeführt. Man erhielt stündlich 300 Gewichtsteile eines Reaktionsaustrages, für dessen Zusammensetzung, wasser- und methanolfrei gerechnet, die folgenden Bestandteile ermittelt wurden:

n-Butanol          0,6 Gew.%
Hydroxibutyraldehyd          < 0,05 Gew.%
Butandiol-1,4          > 98 Gew.%

2-Methylbutandiol-1,4     <0,05 Gew.%
Acetale     < 0,01 Gew.%
Summe Unbekannter     0,1 Gew.%
nicht flüchtige Anteile     0,6 Gew.%

Der Katalysator zeigt selbst nach einer Betriebsdauer von 6 Monaten keinerlei Aktivitätseinbuße.


**Ansprüche**

1. Verfahren zur Herstellung von Alkanolen durch katalytische Hydrierung von Alkinolen mit 3 bis 8 C-Atomen bei höheren Temperaturen und Drücken, dadurch gekennzeichnet, daß man eine Lösung des Alkinols in einem $C_1$-$C_8$-Alkanol mit einem Alkinolgehalt von über 5 Gew.%, einem Wassergehalt von höchstens 10 Gew.% und einem Gehalt an dem Alkanol von 20 bis 85 Gew.%, bei Temperaturen von 100 bis 240°C und Drücken von über 10 bar und einem pH-Wert von größer als 6 hydriert.

2. Verfahren nach Anspruch 1, dadurch gekennzeichnet, daß man im Temperaturbereich von 110°C bis 210°C und bei Drücken von 10 bis 250 bar hydriert.

3. Verfahren nach Anspruch 1, dadurch gekennzeichnet, daß man einen Katalysator verwendet, dessen katalytisch aktive Komponente zu über 50 Gew.% aus Nickel besteht.

4. Verfahren nach Anspruch 1, dadurch gekennzeichnet, daß man als Katalysator das Nickelsalz einer organischen Säure zu der Alkinollösung gibt und im Temperaturbereich von 170 bis 240°C hydriert.

5. Verfahren nach Anspruch 4, dadurch gekennzeichnet, daß man das bei der Hydrierung aus dem Reaktionsgemisch ausgefallene metallische Nickel abtrennt und erneut für die Hydrierung einsetzt.

6. Verfahren nach Anspruch 1, dadurch gekennzeichnet, daß man eine methanolische Lösung von Butin-2-diol-1,4 mit einem Gehalt an Butin-2-diol-1,4 von 30 bis 70 Gew.%, einem Wassergehalt von 0,2 bis 5 Gew.%, einem Methanolgehalt von 30 bis 70 Gew.% und einem Gehalt von 0,2 bis 5 Gew.% an Verunreinigungen, die bei der Synthese von Butin-2-diol-1,4 aus Acetylen und wäßrigem Formaldehyd entstanden sind, hydriert.